# EUROPEAN PATENT SPECIFICATION

(11) **EP 3 906 900 B1**
(45) Date of publication and mention of the grant of the patent: **11.10.2023**
(21) Application number: 20173154.4
(22) Date of filing: 06.05.2020
(51) Int. Cl.: A61F 6/08

(54) **VAGINAL DEVICE FOR PROLAPSE TREATMENT**
VAGINALVORRICHTUNG ZUR BEHANDLUNG VON PROLAPSEN
DISPOSITIF VAGINAL POUR LE TRAITEMENT DU PROLAPSUS

(43) Date of publication of application: 10.11.2021
(73) Proprietor: Stichting Radboud universitair medisch centrum, 6525 GA Nijmegen (NL)
(72) Inventor: NOTTEN, Kim Josephina Bernadette, 6525 GA Nijmegen (NL); WEEMHOFF, Mirjam, 6401 CX Heerlen (NL)
(74) Representative: Nederlandsch Octrooibureau

(56) References cited:
- WO-A1-2018/213187
- WO-A2-2015/054304
- US-A- 183 832
- US-A- 1 750 188
- US-A- 5 323 790
- US-B1- 6 418 930

## Description

### Field of the invention

The present invention relates to a vaginal device for pelvic organ prolapse treatment.

### Background art

International patent publication WO2014/162363 discloses an intravaginal support tool with a balloon including an opening/closing part that can be operated so as to be switched between an open state that lets a fluid pass through and a blocked state that blocks the passage of the fluid. A bag part is present that can be inflated by the inflow of the fluid through the opening/closing part and that can be deflated by the outflow of the fluid through the opening/closing part. Furthermore, an outer bag body is present that houses the bag part of the balloon, and that can be inflated/deflated along with the inflation/deflation of the bag part.

US patent publication US2009/216071 discloses a pessary comprising a main stem and an inflatable bladder disposed about the proximal larger diameter section of the main stem. The main stem further comprises a distal smaller diameter portion having a cap at its distal tip and a check valve disposed there under. The check valve communicates with a central fluid passage that may extend into the proximal larger diameter section of the main stem. Secondary fluid passages connect the central fluid passage to the inflatable bladder volume disposed about the outer circumference of the proximal larger diameter section of the main stem. The main stem provides support to the pelvic structures and the inflatable bladder acts to hold the device in position.

WO2015/054304 A2 discloses the features of the preamble of claim 1.

### Summary of the invention

The present invention seeks to provide a device which is useable as an alternative treatment of pelvic organ prolapse.

According to the present invention, a vaginal device as defined above is provided, comprising an elongated base element, an inflatable element attached to a distal end part of the elongated base element, and a support element attached to a proximal end part of the elongated base element, the support element comprising at least two support members, the at least two support members extending away from the proximal end of the elongated base element. The present invention embodiments relate to a device which has three adaptable parts and which is adjustable in three ways so that it can be altered based on specific patients' characteristics. The length of the elongated base element can be adapted to the vaginal length, the amount of air that is inflated in the inflatable depends on the vaginal space in the depth and the position of the support members can be altered to individual anatomy and support. Furthermore, the present device structure allows to provide a sufficiently small diameter so that insertion and extraction is easier, less painful and thus more patient friendly. As insertion, positioning and removal of the vaginal device is easy, the present invention vaginal device is also very well applicable for self-management by the patient.

### Short description of drawings

The present invention will be discussed in more detail below, with reference to the attached drawings, in which
Fig. 1 shows a top view of an embodiment of the vaginal device according to the present invention;
Fig. 2 shows a bottom view of an embodiment of the vaginal device according to the present invention;
Fig. 3 shows a side view of an embodiment of the vaginal device according to the present invention; and
Fig. 4 shows a cross sectional view of an exemplary implementation of a fluid channel within the vaginal device according to the present invention.

### Description of embodiments

Pelvic Organ Prolapse (POP) is a bothersome condition that occurs in more than half of adult women above the age of forty. The most common and bothersome symptoms of POP are a feeling of pelvic pressure (something is falling out of the vagina), low back pain, painful intercourse or inability to have intercourse, urinary problems such as involuntary release of urine or a frequent or urgent need to urinate and problems with bowel movements such as constipation. These symptoms of POP can have a great negative impact on women's social, physical and psychological well-being. In case of symptomatic POP, treatment options include pessary treatment or surgery.

Pessaries are used for the treatment of POP by more than 85% of gynaecologists and 98% of urogynecologists. Pessary therapy in the standard care consists of pessaries which are available in different shapes and standard sizes. The shapes are often simple and symmetric and do not take the complex shape of the female pelvic bottom into account. Therefore, it is often not possible to find a fitting pessary for women with POP. The ideal fitting pessary should improve the target pelvic floor symptoms, should be comfortable for the patient, should not limit daily activities, should not obstruct voiding or defecation and should not cause vaginal irritation. If a pessary does not properly fit, patients may develop side effects such as blood loss and/or excessive discharge. Furthermore, with the standard pessaries it is often impossible to have sexual intercourse and only a small part of the women with pessary therapy are able to remove/clean and insert the pessary by self-management. Most women find it painful to remove the pessary for fitting or cleaning. The larger part of these women need at least 3-4 doctor visits per year to clean the pessary and insert it again.

Although pessaries have been reported to be effective, the current standard pessary therapy often fails due to improper fit. Because of this improper fit, patients develop side effects such as pain, erosions, excessive vaginal discharge, problems with voiding and defaecation. Quite often it is also impossible to find a proper pessary which fits, meaning that a pessary falls out quite easy during daily activity. Side effects and improper fit causes 20-50% of women to discontinue pessary use within 1 year. The majority of these women decide to undergo surgery. Surgery improves quality of life al lot but the risk of recurrence after prolapse surgery is approximately 30%. A part of the women with prolapse recurrence will start again with pessary therapy. From literature and from clinical practice it is known that after surgery it is even more difficult to find a fitting pessary due to the altered anatomy (i.e. shorter vaginal length). For the prolapse population with a rectocele the standard pessary also doesn't work in most cases with exception of the kubus pessary. The kubus pessary is able to correct a rectoceles but people need to remove it every day or every other day which is quite difficult and stressful for a lot of patients because removing and inserting a kubus pessary can be difficult and painful. If prolapse progresses a new anatomical situation occurs. In these cases it is necessary to find a new pessary and the fitting process starts all over again.

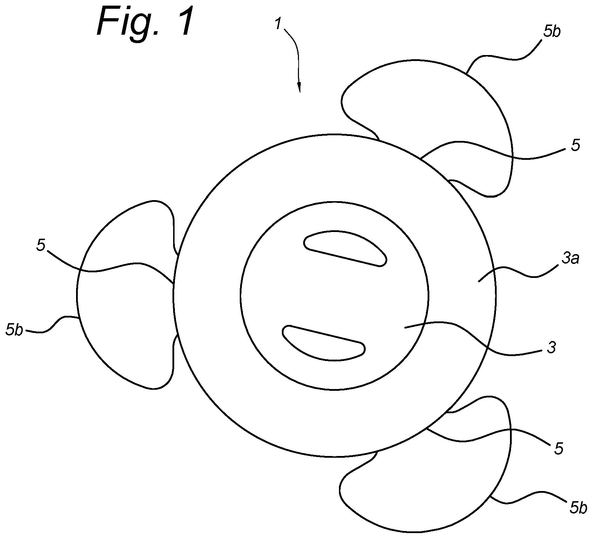

The present invention embodiments address one or more of the aforementioned disadvantages, as will be explained below in relation to several exemplary embodiments. Fig. 1-3 shows a top, bottom and side view, respectively, of an exemplary embodiment of a vaginal device according to the present invention.

A vaginal device 1 is provided for pelvic organ prolapse treatment, comprising an elongated base element 2, an inflatable element 3 attached to a distal end part 2a of the elongated base element 2, and a support element 4 attached to a proximal end part 2b of the elongated base element 3. The support element 4 comprises at least two support members 5, the at least two support members 5 extending away from the proximal end 2b of the elongated base element 2.

The present invention vaginal device 1 can be advantageously used as a therapy for pelvic organ prolapse, and is adjustable to the individual patient in three different manners. The vaginal device 1 is a dynamic device which consist of three adjustable parts 2-4. The first part or inflatable element 3 is to be placed in the fornix posterior, and can be set to the patient specific measurements (vaginal width) and prevents the vaginal device 1 from falling out. Due to this inflatable element 3 peak pressure is prevented and thus less side effects such as discharge and erosion will occur. The inflatable element 3 also corrects the upper parts of the prolapse compartments (uterine prolapse and upper parts of the cystocele and rectocele).

The middle part or elongated base element 2 is patient specific and accounts for the individual vaginal length which can be measured quite easy with the POPQ method (known as such to the skilled person) which is a routine measurement for gynaecologists and experience general practitioners (the mean vaginal length varies between 6 and 10cm). In an embodiment of the present invention, the elongated base element 2 has a length of between 4 and 12 cm. Various sizes of the entire vaginal device 1 are thus possible. As an alternative or additionally, the elongated base element 2 has an adjustable length. This may be implemented by having two or more part mutually moveable and settable parts, or a telescopic construction, e.g. having a middle part to which the distal end part 2a and proximal end part 2b can be attached at various positions.

The last adjustable part is the bottom part or support element 4 of the vaginal device 1. The support element 4 has several (e.g. patient specific) flexible and bendable "legs" or support members 5. The support members 5 can be placed in positions where they maximally support the lower parts of the prolapse for the different compartments. The support members 5 can be radially extending from the longitudinal axis of the elongated base element 2, and/or positioned at an angle to a plane perpendicular to the longitudinal axis.

Due to this adjustable dynamic character of the elements 2-4 of the vaginal device 1, it can be altered to fit each patient with their specific combinations of prolapse including rectocele. Even if the prolapse progresses it is possible to adjust it to a new situation with the same vaginal device 1. Due to this patient specific fit, less side effects occur. The design is also more patient friendly then existing devices because it is easier to insert and extract (by virtue of a small diameter inflatable element 3 when deflated). The present invention embodiments of the vaginal device 1 can replace an existing product utilized in pessary therapy for pelvic organ prolapse treatment. Patient specific (custom made) treatment also ensures cost savings in terms of reduction of outpatient clinic visits, reduction of insertion of improper pessaries (fitting process with standard pessaries which costs 40-80euro each), less side effects and thus less secondary treatment costs, less patients probably will cross over to surgery, and more people will choose this therapy as a primary therapy because it is more patient friendly and there are less side effects.

In a further embodiment one or more components 2, 3, 4 of the vaginal device 1 comprise a biocompatible material. This allows contact of (parts of) the vaginal device 1 with vaginal tissue without causing problems. As an example, silicone materials are used, e.g. having different shore value silicone material types for various elements 2-4, or outer skins of silicone material on the relevant elements 2-4 possibly contacting vaginal tissue.

In an exemplary embodiment, the inflatable element 3 comprises an inflatable ring 3a at an outer circumference of the inflatable element 3. The degree of inflation of the inflatable ring 3a can be varied, allowing to fit the specific patient characteristics, and preventing peak pressure loads to surrounding tissue. In an even further embodiment, the inflatable element 3 (e.g. the inflatable ring 3a part thereof) comprises a (semi-)permeable material. This embodiment has the added advantage that it can be used for regulated administering of a substance, e.g. a fatty substance (anti-adhesive function), a hydrating substance, a substance comprising estrogen (countering atrophy), a substance comprising corticosteroids (countering inflammation), etc.

As shown in the exemplary embodiment of Figs 1-3, the at least two support members 5 may comprise three support members 5. This would provide a good balance between number of components of the vaginal device 1, and efficiency in providing proper support. It is noted that even more than three, e.g. four support members 5 may be provided. By proper selection of materials, the support members 5 may be individually adjusted and positioned after insertion of the vaginal device 1.

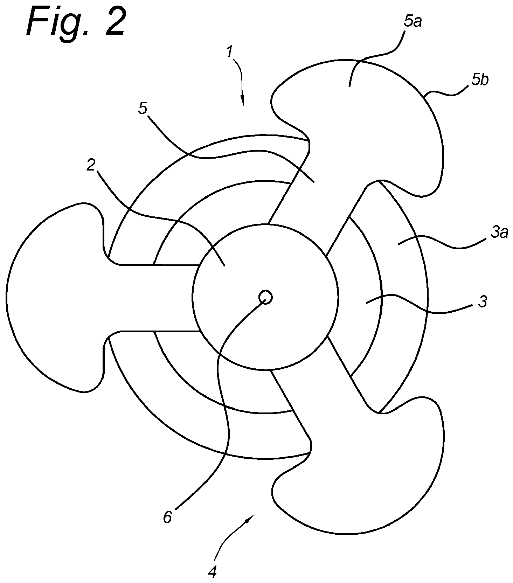

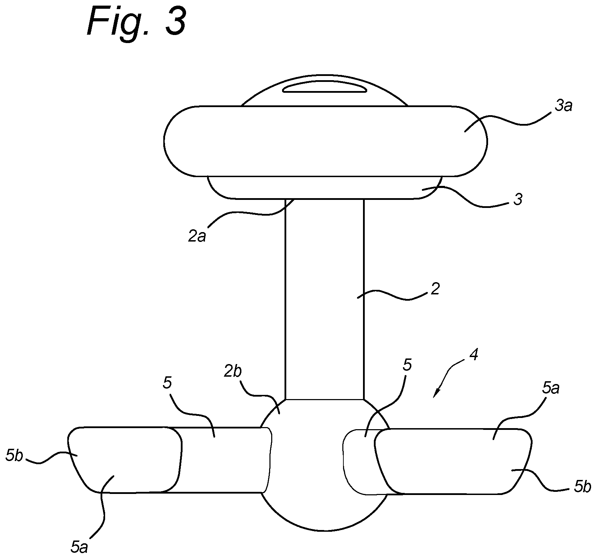

In the bottom view of Fig. 2 and the side view of Fig. 3 it is shown most clearly that, in accordance with a further embodiment of the present invention, the at least two support members 5 comprise an end part 5a having a support surface 5b. This allows to save on material and weight of the vaginal device 1. It is noted that the support surface 5b can then be larger than the (normal) sectional diameter of the support member 5 close to the elongated base element 2. This structure provide for less peak pressure on small internal surfaces during use, i.e. a more even distribution of pressure using the multiple (large) support surfaces 5b.

In even further embodiments, the support surface 5b is provided with a load sensor. The load sensor may be in communication with a software program or app being executed on a computer device such as a smart phone or dedicated monitoring device, via a wired or wireless connection. The software program is e.g. functional to monitor loads over long time periods, in order to assess effectivity of the use of the present invention vaginal device 1, or to generate alarms if certain threshold values are crossed. The software program may have even further alternative or additional functionality, e.g. storing dimensions and other personalized characteristics of the vaginal device 1, storing data on the amount of fluid used or to be used to inflate the inflatable element 3 properly (which can be a varying amount depending on e.g. planned activities, such as sporting or sleeping), providing information to a patient for proper insertion and removal of the vaginal device 1, recording date and time of insertion and removal (or cleaning), etc. When monitoring loads on the support surfaces 5b, recorded data may be analyzed over longer periods and recommendations for adaptation of orientation or position of the support surfaces 5b may be provided.

To ensure the use of the present invention vaginal device 1 is as comfortable to the user as possible, the at least two support members 5 comprise material having a hardness of less than Shore 40A in a further embodiment. To provide sufficient support, the material may also have a hardness of more than Shore 15A. Note that these material characteristics are measured in accordance with ASTM D2240 measurement method, which as such is known to the skilled person.

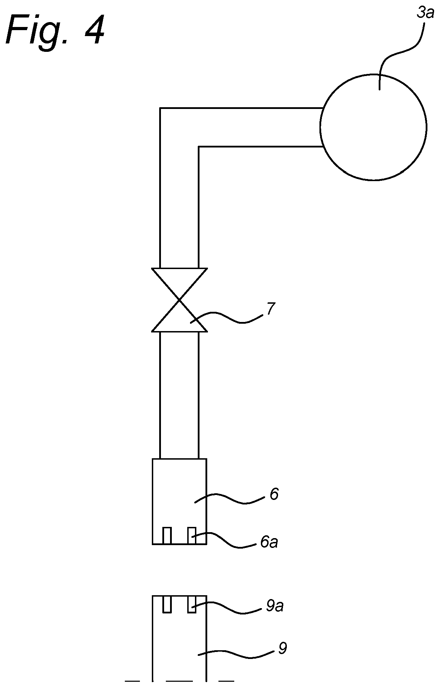

Fig. 4 shows a cross sectional view of an exemplary implementation of a fluid channel within the vaginal device according to the present invention, which is used to inflate or deflate the inflatable element 3 (or more specific, the inflatable ring 3a of the exemplary embodiment shown in Fig. 1-3). In Fig. 4, the inflatable ring 3a is shown, which is in fluid communication with a valve 7 (e.g. using a tube or channel). In other words, in a further embodiment, the vaginal device 1 further comprises a valve 7 in fluid communication with the inflatable element 3. This allows to control the amount of fluid in the inflatable element 3, e.g. by attaching a syringe with fluid (air, water, etc.). The valve 7 is e.g. a one-way valve, allowing the amount of fluid to remain in the inflatable element 3 after removing the syringe. Alternatively, the valve is a pressure sensitive valve, which allows to inflate the inflatable element 3 by applying a high pressure to the valve 7, and to deflate the inflatable element 3 by applying a sufficiently low (or even negative) pressure to the valve 7.

As shown in the cross sectional view of Fig. 4, the elongated base element 2 further comprises a coupling element 6 in fluid communication with inflatable element 3 (e.g. via the valve 7). This allows to use e.g. a filling tube 9 (connected to e.g. a syringe) to inflate/deflate the inflatable element 3. In a further embodiment, the coupling element 6 is positioned in the proximal end part 2b of the elongated base element 2. This allows to connect the tube 9 to the vaginal device 1 in a proper manner, even when the vaginal device 1 is inserted. Furthermore, the valve 7 and the structural connection between coupling element 6 and valve 7 may be arranged such that the valve 7 opens when applying pressure on the coupling element 6, allowing to deflate the inflatable element 3.

As shown in the exemplary embodiment of Fig. 4, the coupling element 6 may be provided with magnets 6a, to allow strong and fluid tight coupling of a filling tube 9 which is also provided with magnets 9a. The magnets 6a, 9a (e.g. strong neodymium magnets) will provide for both guiding the tube 9 towards the coupling element 6, and for a fluid tight coupling. By twisting the filling tube 9, the magnetic connection can be easily disconnected again. The filling tube 9 can also be advantageous when inserting the vaginal device 1, allowing a better grip and control.

As an alternative, or additionally, the filling tube 9 can be provided with an outer diameter which matches with (or is slightly smaller than) the inner diameter of the coupling element 6. Once connected, the filling tube 9 can then be further advanced into the channel connecting coupling element 6 to the inflatable element 3/inflatable ring 3a. The filling tube 9 can furthermore be provided with markings to ensure proper positioning and connection with the channel. In addition, the filling tube may be provided with a separate (e.g. ring shaped) magnet member for coupling to the magnets 6a of the coupling element 6 in the embodiment described in the previous paragraph.

To further enhance the flexibility and usability of the present invention vaginal device 1, the support element 4 comprises an adjustable connector for fixing the position of the support element 4 along the proximal end part 2b of the elongated base element 3. This enables to adjust dimensions of the vaginal device 1 before actual use. The adjustable connector may be implemented in various manner which as such are within the skilled person's knowledge, e.g. using a sliding and locking mechanism.

In an even further embodiment, the support element 4 comprises an actuation mechanism connected to the at least two support members 5. This is e.g. implemented using an unfolding or extending mechanism, which allows to provide a smaller dimension of the entire vaginal device 1, which allows an easier insertion of the vaginal device 1, reverting to an operational position of the support members 5 after insertion. The actuation mechanism may be implemented on basis of mechanical, pneumatic, hydraulic operation, e.g. using resilient material and/or a metal spring.

In an even further embodiment, the vaginal device 1 further comprises an insertion aid, the insertion aid surrounding at least the elongated base element 2 and the support element 4. This furthermore enhances the ease of insertion of the vaginal device 1.

The present invention has been described above with reference to a number of exemplary embodiments as shown in the drawings. Modifications and alternative implementations of some parts or elements are possible, and are included in the scope of protection as defined in the appended claims.

## Claims

1. Vaginal device for pelvic organ prolapse treatment, comprising:
an elongated base element (2),
an inflatable element (3) for placement in the fornix posterior and attached to a distal end part (2a) of the elongated base element (2), and
a support element (4) attached to a proximal end part (2b) of the elongated base element (3),
the support element (4) comprising at least two support members (5),
the at least two support members (5) extending away from the proximal end (2b) of the elongated base element (2), wherein the at least two support members (5) are arranged to support lower parts of prolapsed compartments, and
**characterized in that**
the support element (4) comprises an adjustable connector for fixing the position of the support element (4) along the proximal end part (2b) of the elongated base element (3).

2. Vaginal device according to claim 1, wherein the inflatable element (3) comprises an inflatable ring (3a) at an outer circumference of the inflatable element (3).

3. Vaginal device according to claim 1 or 2, further comprising a valve (7) in fluid communication with the inflatable element (3).

4. Vaginal device according to any one of claims 1-3, wherein the elongated base element (2) further comprises a coupling element (6) in fluid communication with the inflatable element (3).

5. Vaginal device according to claim 4, wherein the coupling element (6) is positioned in the proximal end part (2b) of the elongated base element (2).

6. Vaginal device according to any one of claims 1-5, wherein the inflatable element (3) comprises a permeable material.

7. Vaginal device according to any one of claims 1-6, wherein the at least two support members (5) comprise three support members (5).

8. Vaginal device according to any one of claims 1-7, wherein the at least two support members (5) comprise an end part (5a) having a support surface (5b).

9. Vaginal device according to claim 8, wherein the support surface (5b) is provided with a load sensor.

10. Vaginal device according to any one of claims 1-9, wherein the at least two support members (5) comprise material having a hardness of less than Shore 40A.

11. Vaginal device according to any one of claims 1-10, wherein the support element (4) comprises an actuation mechanism connected to the at least two support members (5)..

12. Vaginal device according to any one of claims 1-11, wherein the elongated base element (2) has a length of between 4 and 12 cm.

13. Vaginal device according to any one of claims 1-12, further comprising an insertion aid, the insertion aid surrounding at least the elongated base element (2) and the support element (4).

14. Vaginal device according to any one of claims 1-13, wherein one or more components (2, 3, 4) of the vaginal device (1) comprise a biocompatible material.

## Patentansprüche

1. Vaginalvorrichtung zur Behandlung von Beckenorganprolapsen, umfassend:
ein längliches Basiselement (2),
ein aufblasbares Element (3) zur Platzierung im posterioren Fornix und angebracht an einem distalen Endteil (2a) des länglichen Basiselements (2), und
ein Stützelement (4), das an einem proximalen Endteil (2b) des länglichen Basiselements (2) angebracht ist,
wobei das Stützelement (4) mindestens zwei Stützglieder (5) umfasst,
wobei sich die mindestens zwei Stützglieder (5) von dem proximalen Ende (2b) des länglichen Basiselements (2) weg erstrecken, wobei die mindestens zwei Stützglieder (5) eingerichtet sind, um untere Teile der prolabierten Kompartments zu stützen, und
**dadurch gekennzeichnet, dass** das Stützelement (4) einen einstellbaren Verbinder zum Fixieren der Position des Stützelements (4) entlang des proximalen Endteils (2b) des länglichen Basiselements (2) umfasst.

2. Vaginalvorrichtung nach Anspruch 1, wobei das aufblasbare Element (3) einen aufblasbaren Ring (3a) an einem Außenumfang des aufblasbaren Elements (3) umfasst.

3. Vaginalvorrichtung nach Anspruch 1 oder 2, ferner umfassend ein Ventil (7) in Fluidkommunikation mit dem aufblasbaren Element (3).

4. Vaginalvorrichtung nach einem der Ansprüche 1 bis 3, wobei das längliche Basiselement (2) ferner ein Kupplungselement (6) in Fluidkommunikation mit dem aufblasbaren Element (3) umfasst.

5. Vaginalvorrichtung nach Anspruch 4, wobei das Kupplungselement (6) in dem proximalen Endteil (2b) des länglichen Basiselements (2) positioniert ist.

6. Vaginalvorrichtung nach einem der Ansprüche 1 bis 5, wobei das aufblasbare Element (3) ein permeables Material umfasst.

7. Vaginalvorrichtung nach einem der Ansprüche 1 bis 6, wobei die mindestens zwei Stützglieder (5) drei Stützglieder (5) umfassen.

8. Vaginalvorrichtung nach einem der Ansprüche 1 bis 7, wobei die mindestens zwei Stützglieder (5) einen Endteil (5a) mit einer Stützfläche (5b) umfassen.

9. Vaginalvorrichtung nach Anspruch 8, wobei die Stützfläche (5b) mit einem Lastsensor versehen ist.

10. Vaginalvorrichtung nach einem der Ansprüche 1 bis 9, wobei die mindestens zwei Stützglieder (5) Material mit einer Härte von weniger als Shore 40A umfassen.

11. Vaginalvorrichtung nach einem der Ansprüche 1 bis 10, wobei das Stützelement (4) einen Betätigungsmechanismus umfasst, der mit den mindestens zwei Stützgliedern (5) verbunden ist.

12. Vaginalvorrichtung nach einem der Ansprüche 1 bis 11, wobei das längliche Basiselement (2) eine Länge zwischen 4 und 12 cm aufweist.

13. Vaginalvorrichtung nach einem der Ansprüche 1 bis 12, ferner umfassend eine Einsetzhilfe, wobei die Einsetzhilfe mindestens das längliche Basiselement (2) und das Stützelement (4) umgibt.

14. Vaginalvorrichtung nach einem der Ansprüche 1 bis 13, wobei eine oder mehrere Komponenten (2, 3, 4) der Vaginalvorrichtung (1) ein biokompatibles Material umfassen.

## Revendications

1. Dispositif vaginal pour un traitement de prolapsus d'organe pelvien, comprenant :
un élément de base allongé (2),
un élément gonflable (3) destiné à être placé dans le fomix postérieur et fixé à une partie d'extrémité distale (2a) de l'élément de base allongé (2), et
un élément de support (4) fixé à une partie d'extrémité proximale (2b) de l'élément de base allongé (3),
l'élément de support (4) comprenant au moins deux membres de support (5),
les au moins deux membres de support (5) s'étendant à partir de l'extrémité proximale (2b) de l'élément de base allongé (2), dans lequel les au moins deux membres de support (5) sont agencés pour maintenir des parties inférieures des compartiments à prolapsus, et
**caractérisé en ce que** l'élément de support (4) comprend un connecteur réglable pour fixer la position de l'élément de support (4) le long de la partie d'extrémité proximale (2b) de l'élément de base allongé (3).

2. Dispositif vaginal selon la revendication 1, dans lequel l'élément gonflable (3) comprend un anneau gonflable (3a) au niveau d'une circonférence extérieure de l'élément gonflable (3).

3. Dispositif vaginal selon la revendication 1 ou 2, comprenant en outre une valve (7) en communication fluidique avec l'élément gonflable (3).

4. Dispositif vaginal selon l'une quelconque des revendications 1 à 3, dans lequel l'élément de base allongé (2) comprend en outre un élément de couplage (6) en communication fluidique avec l'élément gonflable (3).

5. Dispositif vaginal selon la revendication 4, dans lequel l'élément de couplage (6) est positionné dans la partie d'extrémité proximale (2b) de l'élément de base allongé (2).

6. Dispositif vaginal selon l'une quelconque des revendications 1 à 5, dans lequel l'élément gonflable (3) comprend un matériau perméable.

7. Dispositif vaginal selon l'une quelconque des revendications 1 à 6, dans lequel les au moins deux membres de support (5) comprennent trois membres de support (5).

8. Dispositif vaginal selon l'une quelconque des revendications 1 à 7, dans lequel les au moins deux membres de support (5) comprennent une partie d'extrémité (5a) présentant une surface de support (5b).

9. Dispositif vaginal selon la revendication 8, dans lequel la surface de support (5b) est pourvue d'un capteur de charge.

10. Dispositif vaginal selon l'une quelconque des revendications 1 à 9, dans lequel les au moins deux éléments de support (5) comprennent un matériau présentant une dureté inférieure à 40 shore A.

11. Dispositif vaginal selon l'une quelconque des revendications 1 à 10, dans lequel l'élément de support (4) comprend un mécanisme d'actionnement relié aux au moins deux membres de support (5).

12. Dispositif vaginal selon l'une quelconque des revendications 1 à 11, dans lequel l'élément de base allongé (2) présente une longueur comprise entre 4 et 12 cm.

13. Dispositif vaginal selon l'une quelconque des revendications 1 à 12, comprenant en outre un auxiliaire d'insertion, l'auxiliaire d'insertion entourant au moins l'élément de base allongé (2) et l'élément de support (4).

14. Dispositif vaginal selon l'une quelconque des revendications 1 à 13, dans lequel un ou plusieurs composants (2, 3, 4) du dispositif vaginal (1) comprennent un matériau biocompatible.
